# EUROPEAN PATENT SPECIFICATION

(11) **EP 1 811 001 B1**
(45) Date of publication and mention of the grant of the patent: **11.07.2012**
(21) Application number: 07001141.6
(22) Date of filing: 02.06.2000
(51) Int. Cl.: C12N 9/02, C09D 5/16

(54) **Anti-fouling composition**
Bewuchsverhindernde Zusammensetzung
Composition antisalissures

(30) Priority: 04.06.1999 GB 9913050
(43) Date of publication of application: 25.07.2007
(62) Divisional of application: 00935439.0
(73) Proprietor: Dupont Nutrition Biosciences ApS, 1001 Copenhagen K (DK)
(72) Inventor: Poulsen, Charlotte, Horsmans, 8220 Braband (DK); Kragh, Karsten, Matthias, 8260 Viby J (DK)
(74) Representative: Simcox, Michael Thomas

(56) References cited:
- EP-A- 0 639 600
- EP-A- 0 866 103
- US-A- 4 297 137
- PATENT ABSTRACTS OF JAPAN vol. 014, no. 539 (C-0782), 28 November 1990 (1990-11-28) & JP 02 227465 A (DAINIPPON INK & CHEM INC;OTHERS: 01), 10 September 1990 (1990-09-10)

## Description

The present invention relates to an anti-fouling composition. In particular, the present invention relates to an anti-fouling composition comprising an enzyme capable of producing a compound having an anti-fouling effect.

As discussed in US-A-5071479 biocides are required in many different environments, such as antifungal agents in house paints, fresh water algicides, and anti-fouling agents for marine structures exposed to sea water flora and fauna. As is known, mildew or fungus may grow on house paints and the like, and utilizes the paint medium as a nutrient, or in some cases, the underlying substrate, such as wood, as the nutrient. For obvious reasons, this may cause damage to the painted surface and/or a deterioration in the appearance of the painted surface. A biocide may be incorporated in the paint and when the mycelia and fruiting bodies of the fungi contact or penetrate the paint film and thus, through intimate contact with the biocide in the film, the fungi are destroyed. In cooling towers utilizing fresh water, slimes, mould and algae may develop if effective compounds for combating their growth are not present.

As discussed in US-A-5071479 the growth of marine organisms on the submerged parts of a ship's hull is a particular problem. Such growth increases the frictional resistance of the hull to passage through water, leading to increased fuel consumption and/or a reduction in the speed of the ship. Marine growths accumulate so rapidly that the remedy of cleaning and repainting as required in dry-dock is generally considered too expensive. An alternative which has been practiced with increasing efficiency over the years, is to limit the extent of fouling by applying to the hull a top coat paint incorporating anti-fouling agents. The anti-fouling agents are biocides which are freed from the surface of the paint over a period of time at a concentration lethal to marine organisms at the hull surface. The anti-fouling paint fails only when the concentration of biocide available at the paint surface falls below the lethal concentration and with modem paints up to two years of useful life is expected.

An extremely widely used biocide, particularly in marine anti-fouls, is tributyl tin (TBT). However, there is a growing concern about the environmental effects caused by using such organic tin biocides at their present commercial levels as an anti-foulant active ingredient in coating compositions for aquatic (marine) applications. It has been shown that, due to the wide-spread use of tributyltin-type compounds in particular, at concentrations as high as 20 wt.% in paints for ship bottoms, the pollution of surrounding water due to leaching has reached such a level as to cause the degradation of mussel and shell organisms. These effects have been detected along the French-British coastline and a similar effect has been confirmed in U.S. and Far East waters. Under the most recent regulatory restrictions, with limited exceptions, pleasure boats up to 25 meters long are no longer permitted to use anti-foulant paint containing high levels of tributyltin compounds.

Research has shown that as long as the leaching rate of tin can be maintained at or below about 4 µg/cm² per day, aquatic life does not appear to be affected over the long term. However, it has also been found that to be effective for controlling marine algae, as well as higher developed marine organisms, from the painted surface of ship bottoms, a certain minimum leaching rate of tin of about 9 to 16 µg/cm²/day is required. Usually, this higher leaching rate is achieved with a concentration of tributyltin compound at about 15% to 20% by weight of paint.

In view of the effectiveness of TBT regulatory authorities have reluctantly agreed that as long as there is no satisfactory substitute for the anti-foulant organic tin active ingredients, larger ships, i.e., those above a length of 25 meters, are still permitted to use such compounds to minimize fouling. There is therefore a desire to provide alternative biocides to TBT based compounds.

US-A-4297137 discloses that the effects of an anti-fouling composition can be lengthened by moderating the release of the anti-fouling constituents. This document discloses anti-fouling paints comprising at least one substance toxic to marine organism uniformly incorporated into a discontinuous solid matrix which is insoluble in sea water and is dispersed in the paint. The matrix is at least partially formed from at least one substance which becomes soluble in sea water under the action of enzymes liberated by the marine organisms to be inhibited and/or by the bacterial film in contact with the paint. Thus when marine organism become associated with the painted surface, the toxic substance is released and the organisms inhibited. Similar to prior art disclosures, the toxic substances envisaged by US-A-4297137 include only the well known copper and tin based compounds, such as TBT.

Abarzua et al., Mar. Ecol. Prog. Ser., Vol. 123: 301-312, 1995 "Biotechnological investigation for the prevention of biofouling. L Biological and biochemical principles for the prevention of biofouling" propose the extraction of biogenic agents having antibacterial, anti-algal, anti-protozoan and anti-macrofouling properties from algae and marine invertebrates. It is proposed that the structure of the extracted agents may be determined, subsequently synthesised and the synthesised agent used in the prevention of biofouling. No teaching of the extraction or of the synthesis is provided.

EP-A-0866103 discloses a method for controlled release of compounds having antimicrobial activity and coating compositions utilising this system. The method comprises the incorporation of an enzyme and a substrate into a matrix. The enzyme acts of the substrate to provide a compound. In an envisaged embodiment the compound may be acted on by a further enzyme. The substrate and enzyme(s) produce a compound having antimicrobial activity.

US-A-5747078 relates to food products. The document teaches that microbial contamination of food and feed, which can cause severe health problems, may be inhibited by a composition comprising a lactoperoxidase system which provides for the sustained release of hydrogen peroxide. The hydrogen peroxide is prepared by the reaction of an oxidoreductase with an oxidisable substrate. The hydrogen peroxide then reacts with thiocyanate, under catalysis by lactoperoxidase, to produce hypothiocyanate. The hypothiocyanate may then act as an anti-microbial agent. This document provides a background teaching of immobilised enzyme systems. The document is silent concerning anti-foulants or any micro-organism which results in fouling properties.

The present invention alleviates the problem of the prior art

Aspects of the present invention are defined in the appended claims. These and other preferred aspects are discussed below.

It has been found that the provision of an integrated system for the generation of an anti-fouling compound utilising an enzyme from a marine organism provides a stable system which
- has long term effectiveness in harsh environment such as marine environments
- requires less substrate than prior art systems to provide a given anti-microbial effect. Enzymes from marine organisms, such as algal hexose oxidase (HOX), have low Km values for glucose, namely 2.7 mM. This low Km means that the enzyme has a very high affinity for glucose. In contrast non-marine enzymes such as non-marine glucose oxidase (GOX) may have at least ten fold higher Km value for glucose. In other words prior enzyme systems have a much lower affinity for glucose than that of the present invention. In antifouling applications, this will give a significant difference, since an enzyme with high glucose affinity will be able to convert all the glucose present. On the other hand an enzyme with lower glucose affinity is anticipated to allow leaching of glucose to the surrounding environment. Leaching of glucose will counter the desired antifouling activity because glucose will be a substrate for fouling organisms. Leaching of glucose will therefore lead to increased fouling.
- requires less enzyme than prior art systems to provide a given anti-microbial effect. Enzymes from marine organisms, such as algal HOX, also have low Km value for oxygen, again lower than the Km value for oxygen of prior art systems such as GOX. Again this higher affinity for the substrate gives algal HOX an advantage. This is because in antifouling applications the worst fouling will be in "closed" environments like harbours with low waterexchange and high growth of algae and other fouling organisms. At exactly these places were the fouling is worst, the oxygen content of the water will also be the lowest compared to open sea. Enzymes from marine organisms with high affinity for oxygen will therefore be advantageous.
- provides improved activity at likely operational temperatures.
   In surface coating compositions such as antifouling paint the anti-microbial (antifouling) compound typically has to be active between 15 and 30°C. For anti-fouling compositions this is the seawater temperature where fouling gives a problem. Contrary to prior art systems, enzymes from marine organisms, such as algal HOX, have optimum temperature of activity exactly at the optimum temperature for fouling and these enzymes are therefore perfectly suited as an antifouling agent. The optimum temperature is shown in Example 9
- utilises safe and readily available substrates.
- has improved salt tolerance which leads to further improved activity in marine environments.
- is resistant to degradation by fouling organisms
   enzymes from marine organisms, such as algal HOX, are remarkably protease resistant enzymes. They will survive treatment with pronase (a broad spectrum protease preparation) without any loss of activity. This protease resistance is considered especially important in the antifouling application since the enzyme therefore will be resistant to degradation by proteases from the antifouling organisms which are trying to attach themselves onto the coated surface.

In the present specification "foulants" referred to by the terms "anti-foul(s)", "anti-fouling", and "anti-foulants" include organisms which may reside and/or grow on the surface to be treated with the present composition. The organisms include microorganisms such as bacteria, fungi and protozoa, and algae and organisms such as algae, plants and animals. The organism may be marine organisms.

The composition of the present invention comprises a precursor enzyme and a precursor substrate, wherein the precursor enzyme and the precursor substrate generate a substrate for the enzyme of the present invention by action of the precursor enzyme on the precursor substrate. This combination of precursor enzyme and precursor substrate is herein after referred to as "substrate generator".

The enzyme of the present system may be obtained or may be obtainable from a marine micro-organism

Preferably the enzyme of the present system is obtained or is obtainable from a marine alga. Preferably the enzyme of the present system is obtained or is obtainable from *Chondrus cripus.*

Preferably, the anti-fouling compound is hydrogen peroxide.

Preferably, the enzyme is an oxidase..Preferably, the enzyme is selected from glucose oxidase, L amino acid oxidase, D amino oxidase, galactose oxidase, hexose oxidase, pyranose oxidase, malate oxidase, cholesterol oxidase, arylalcohol oxidase, alcohol oxidase, lathosterol oxidase, aspartate oxidase, amine oxidase, D glutamate oxidase, ethanolamine oxidase, NADH oxidase, urate oxidase (uricase) and mixtures thereof. Preferably, the enzyme is hexose oxidase.

### HEXOSE OXIDASE (HOX) ENZYME

Hexose oxidase (D-hexose: O₂-oxidoreductase, EC 1.1.3.5) (also referred to as HOX) is an enzyme that in the presence of oxygen is capable of oxidising D-glucose and several other reducing sugars including maltose, lactose and cellobiose to their corresponding lactones with subsequent hydrolysis to the respective aldobionic acids. Accordingly, HOX differs from another oxidoreductase, glucose oxidase, which can only convert D-glucose, in that the enzyme can utilise a broader range of sugar substrates. The oxidation catalysed by HOX can be illustrated as follows:

D-glucose + O₂ ---> γ-D-gluconolactone + H₂O₂,

or

D-galactose + O₂ ---> γ-D-galactonolactone + H₂O₂

HOX is produced naturally by several marine algal species. Such species are found *inter alia* in the family *Gigartinaceae.* As used herein, the term "HOX" denotes an enzyme which is capable of oxidising the substrates selected from the group consisting of D-glucose, D-galactose, D-mannose, maltose, lactose and cellobiose.

Preferably, the hexose oxidase is obtainable or is obtained from marine algae *Chondrus cripus.*

In one aspect the hexose oxidase enzyme is an enzyme covered by the disclosure of EP-A-0832245

### HEXOSE OXIDASE (HOX) PRODUCTION

The gene encoding the HOX enzyme has been cloned from the marine algae *Chondrus crispus* (Stougaard and Hansen 1996, Hansen and Stougaard, 1997). The methylotrophic yeast *Hansenula polymorpha* (developed at Rhein Biotech, Dusseldorf/Germany as an expression system for heterologous proteins) has also been used to produce the HOX enzyme (the native protein was purified from marine algae (Poulsen and Høstrup, 1998)). WO 96/40935 and WO 98/13478 also disclose the cloning and expression in recombinant host organisms of a gene encoding a protein with HOX activity.

In a preferred embodiment, the hexose oxidase enzyme comprises the amino acid sequence set out in SEQ ID No 1 or a variant, homologue, derivative or fragment thereof. In a preferred embodiment, the hexose oxidase enzyme comprises the amino acid sequence set out in SEQ ID No 1.

In a preferred embodiment, the hexose oxidase enzyme is encoded by a nucleotide sequence set out in SEQ ID No 1 or a variant, homologue, derivative or fragment thereof. In a preferred embodiment, the hexose oxidase enzyme is encoded by a nucleotide sequence set out in SEQ ID No 1.

In a preferred embodiment, the hexose oxidase enzyme is encoded by a nucleotide sequence capable of hybridising to the nucleotide sequence set out in SEQ ID No 1 or a variant, homologue, derivative or fragment thereof or a sequence complementary to the hybridisable sequence. In a preferred embodiment, the hexose oxidase enzyme is encoded by a nucleotide sequence capable of hybridising to the nucleotide sequence set out in SEQ ID No 1 or a sequence complementary to the hybridisable sequence.

The enzyme, preferably the hexose oxidase enzyme may be prepared in a manner described in British Patent Application No. 9927801.2

### VARIANTS/HOMOLOGUES/DERIVATIVES (AMINO ACID SEQUENCE)

Preferred amino acid sequences of the present invention are set out in SEQ ID No 1 or are sequences obtainable from the HOX enzyme of the present invention but also include homologous sequences obtained from any source, for example related viral/bacterial proteins, cellular homologues and synthetic peptides, as well as variants or derivatives thereof.

Thus, the present invention covers variants, homologues or derivatives of the amino acid sequences presented herein, as well as variants, homologues or derivatives of the nucleotide sequence coding for those amino acid sequences.

In the context of the present invention, a homologous sequence is taken to include an amino acid sequence which is at least 75, 85 or 90% identical, preferably at least 95 or 98% identical at the amino acid level over at least, for example, the amino acid sequence as set out in SEQ ID No 1 of the sequence listing herein. In particular, homology should typically be considered with respect to those regions of the sequence known to be essential for enzyme activity rather than non-essential neighbouring sequences. These regions include but are not limited to the putative FAD binding domains in HOX such as SGGH₇₉C, LGGH₁₄₆I and LGGH₃₂₀A. Although homology can also be considered in terms of similarity (i.e. amino acid residues having similar chemical properties/functions), in the context of the present invention it is preferred to express homology in terms of sequence identity.

Homology comparisons can be conducted by eye, or more usually, with the aid of readily available sequence comparison programs. These commercially available computer programs can calculate % homology between two or more sequences.

% homology may be calculated over contiguous sequences, i.e. one sequence is aligned with the other sequence and each amino acid in one sequence is directly compared with the corresponding amino acid in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion will cause the following amino acid residues to be put out of alignment, thus potentially resulting in a large reduction in % homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example when using the GCG Wisconsin Bestfit package (see below) the default gap penalty for amino acid sequences is -12 for a gap and -4 for each extension.

Calculation of maximum % homology therefore firstly requires the production of an optimal alignment, taking into consideration gap penalties. A suitable computer program for carrying out such an alignment is the GCG Wisconsin Bestfit package (University of Wisconsin, U.S.A.; Devereux et al., 1984, Nucleic Acids Research 12:387). Examples of other software than can perform sequence comparisons include, but are not limited to, the BLAST package (see Ausubel *et al.,* 1999 *ibid -* Chapter 18), FASTA (Atschul et al., 1990, J. Mol. Biol., 403-410) and the GENEWORKS suite of comparison tools. Both BLAST and FASTA are available for offline and online searching (see Ausubel et al., 1999 ibid, pages 7-58 to 7-60). However it is preferred to use the GCG Bestfit program.

Although the final % homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table if supplied (see user manual for further details). It is preferred to use the public default values for the GCG package, or in the case of other software, the default matrix, such as BLOSUM62.

Once the software has produced an optimal alignment, it is possible to calculate % homology, preferably % sequence identity. The software typically does this as part of the sequence comparison and generates a numerical result.

The terms "variant" or "derivative" in relation to the amino acid sequences of the present invention includes any substitution of, variation of, modification of, replacement of, deletion of or addition of one (or more) amino acids from or to the sequence providing the resultant amino acid sequence has an enzyme activity, preferably having at least the same enzyme activity as the amino acid sequence set out in SEQ ID No 1.

SEQ ID No 1 may be modified for use in the present invention. Typically, modifications are made that maintain the enzyme activity of the sequence. Amino acid substitutions may be made, for example from 1, 2 or 3 to 10 or 20 substitutions provided that the modified sequence retains the required enzyme activity. Amino acid substitutions may include the use of non-naturally occurring analogues.

SEQ ID No 1 of the present invention may also have deletions, insertions or substitutions of amino acid residues which produce a silent change and result in a functionally equivalent enzyme. Deliberate amino acid substitutions may be made on the basis of similarity in polarity, charge, solubility, hydrophobicity, hydrophilicity, and/or the amphipathic nature of the residues as long as the enzyme activity of the HOX enzyme is retained. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine.

Conservative substitutions may be made, for example according to the Table below. Amino acids in the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |

### VARIANTS/HOMOLOGUES/DERIVATIVES (NUCLEOTIDE SEQUENCE)

It will be understood by a skilled person that numerous different nucleotide sequences can encode the same HOX enzyme as a result of the degeneracy of the genetic code. In addition, it is to be understood that skilled persons may, using routine techniques, make nucleotide substitutions that do not affect the HOX enzyme encoded by the nucleotide sequence of the invention to reflect the codon usage of any particular host organism in which the HOX enzyme of the present invention is to be expressed.

The terms "variant", "homologue" or "derivative" in relation to the nucleotide sequence set out in SEQ ID No 1 of the present invention includes any substitution of, variation of, modification of, replacement of, deletion of or addition of one (or more) nucleic acid from or to the sequence providing the resultant nucleotide sequence codes for a HOX enzyme having an enzyme activity, preferably having at least the same activity as the nucleotide sequence set out in SEQ ID No 1 of the sequence listings.

As indicated above, with respect to sequence homology, preferably there is at least 75%, more preferably at least 85%, more preferably at least 90% homology to the sequences shown in the sequence listing herein. More preferably there is at least 95%, more preferably at least 98%, homology. Nucleotide homology comparisons may be conducted as described above. A preferred sequence comparison program is the GCG Wisconsin Bestfit program described above. The default scoring matrix has a match value of 10 for each identical nucleotide and -9 for each mismatch. The default gap creation penalty is - 50 and the default gap extension penalty is -3 for each nucleotide.

The present invention also encompasses nucleotide sequences that are capable of hybridising selectively to the sequences presented herein, or any variant, fragment or derivative thereof, or to the complement of any of the above. Nucleotide sequences are preferably at least 15 nucleotides in length, more preferably at least 20, 30, 40 or 50 nucleotides in length.

### SUBSTRATE

Preferably, the substrate is selected from peptides, L amino acid, and carbohydrates/sugars, including hexoses, preferably glucose, galactose, lactose, 2-deoxyglucose, pyranose, xylan, cellulose, inulin, starch, dextran, pectin, and mixtures thereof.

In a highly preferred embodiment the enzyme/substrate combination is selected from glucose/hexose oxidase, glucose/glucose oxidase, L amino acid/L amino acid oxidase, galactose/galactose oxidase, lactose/β-galactosidase/hexose oxidase, lactose/β-galactosidase/glucose oxidase, 2-deoxyglucose/glucose oxidase, pyranose/pyranose oxidase, and mixtures thereof.

In one aspect the anti-foulant compound is generated by action of the enzyme on the substrate which is present in the composition. Thus the anti-foulant compound is generated by a "one-step" process. In some cases the substrate may be prepared in situ. In these cases, the composition further comprises a precursor enzyme and a precursor substrate wherein the precursor enzyme and the precursor substrate are selected such that the precursor enzyme generates the substrate. In this latter aspect the anti-foulant compound is generated by a "two-step" process

In the one-step process preferably the enzyme is selected from hexose oxidase, glucose oxidase, L amino acid oxidase, galactose oxidase, pyranose oxidase, and mixtures thereof.

In the one-step process preferably the substrate is selected from a hexose, preferably glucose, L amino acid, galactose, 2-deoxyglucose, pyranose, and mixtures thereof.

In the one-step process preferably the enzyme/substrate combination is selected from glucose/hexose oxidase, glucose/glucose oxidase, L amino acid/L amino acid oxidase, galactose/galactose oxidase, 2-deoxyglucose/glucose oxidase, pyranose/pyranose oxidase, and mixtures thereof.

In the two-step process preferably the enzyme is hexose oxidase.

In the two-step process preferably the substrate is glucose.

In the two-step process preferably the precursor enzyme is amyloglucosidase.

In the two-step process preferably the precursor substrate is starch.

Thus in the two-step process preferably the precursor substrate/precursor enzyme/enzyme combination is starch/amyloglucosidase/hexose oxidase.

Preferably, the precursor substrate of the two-step process is selected from oligomers and polymers of substrates for oxidative enzymes, starch, lactose, cellulose, dextrose, peptide, inulin, and mixtures thereof.

The provision of precursor substrates are particularly preferred because they provide for sustained and/or prolonged release of substrate by action of the precursor enzyme on the precursor substrate.

Native starch is particularly preferred as a precursor substrate. Native starch provides densely packed crystals which can be readily applied in a surface coatings. Moreover, Native starch is water insoluble.

Cellulose is also a particularly preferred as a precursor substrate. Cellulose is a common component in paint and use of cellulose a precursor substrate reduces the number of additional components which must be added to a paint composition.

Preferably, the precursor enzyme of the two-step process is selected from exo-acting enzymes capable of degrading oligomeric or polymeric substrates to monomeric units, e.g. β-galactosidase, peptidase; amyloglucosidase, and mixtures thereof.

Optionally, the composition further comprises a binder to immobilise at least one of the constituents, optionally to immobilise the enzymes.

The compositions of the present invention may be formulated as coatings, lacquers, stains, enamels and the like, hereinafter referred to generically as "coating(s)".

Thus, in one aspect the present invention provides a coating consisting of a composition as defined above.

Preferably, the coating is formulated for treatment of a surface selected from outdoor wood work, external surface of a central heating system, and a hull of a marine vessel.

The coating may include a liquid vehicle (solvent) for dissolving or suspending the composition.

The liquid vehicle may be selected from any liquid which does not interfere with the activities of any essential components of the composition. In particular, the liquid vehicle should not interfere with the activity of the essential enzyme(s) and/or anti-foulant compound. Suitable liquid vehicles are disclosed in US-A-5071479 and include water and organic solvents including aliphatic hydrocarbons, aromatic hydrocarbons, such as xylene, toluene, mixtures of aliphatic and aromatic hydrocarbons having boiling points between 100 and 320°C, preferably between 150 and 230°C; high aromatic petroleum distillates, e.g., solvent naptha, distilled tar oil and mixtures thereof; alcohols such as butanol, octanol and glycols; vegetable and mineral oils; ketones such as acetone; petroleum fractions such as mineral spirits and kerosene, chlorinated hydrocarbons, glycol esters, glycol ester ethers, derivatives and mixtures thereof.

The liquid vehicle may contain at least one polar solvent, such as water, in admixture with an oily or oil-like low-volatility organic solvent, such as the mixture of aromatic and aliphatic solvents found in white spirits, also commonly called mineral spirits.

The vehicle may typically contain at least one of a diluent, an emulsifier, a wetting agent, a dispersing agent or other surface active agent. Examples of suitable emulsifiers are disclosed in US-A-5071479 and include nonylphenol-ethylene oxide ethers, polyoxyethylene sorbitol esters or polyoxyethylene sorbitan esters of fatty acids, derivatives and mixtures thereof.

Any suitable surface coating material may be incorporated in the composition and/or coating of the present invention. Examples of trade-recognized coating materials are polyvinyl chloride resins in a solvent based system, chlorinated rubbers in a solvent based system, acrylic resins and methacrylate resins in solvent based or aqueous systems, vinyl chloride-vinyl acetate copolymer systems as aqueous dispersions or solvent based systems, butadiene copolymers such as butadiene-styrene rubbers, butadiene-acrylonitrile rubbers, and butadiene-styrene-acrylonitrile rubbers, drying oils such as linseed oil, alkyd resins, asphalt, epoxy resins, urethane resins, polyester resins, phenolic resins, derivatives and mixtures thereof.

The composition and/or coating of the present invention may contain pigments selected from inorganic pigments, such as titanium dioxide, ferric oxide, silica, talc, or china clay, organic pigments such as carbon black or dyes insoluble in sea water, derivatives and mixtures thereof.

The composition and/or coating of the present invention may contain materials such as rosin to provide controlled release of the anti-foulant compound, rosin being to a very slight extent soluble in sea water.

The composition and/or coating of the present invention may contain plasticisers, rheology characteristic modifiers, other conventional ingredients and mixtures thereof.

The composition and/or coating of the present invention, particularly the coating, further comprise an adjuvant conventionally employed in compositions used for protecting materials exposed to an aquatic environment. These adjuvants may be selected from additional fungicides, auxiliary solvents, processing additives such as defoamers, fixatives, plasticisers, UV-stabilizers or stability enhancers, water soluble or water insoluble dyes, color pigments, siccatives, corrosion inhibitors, thickeners or anti-settlement agents such as carboxymethyl cellulose, polyacrylic acid or polymethacrylic acid, anti-skinning agents, derivatives and mixtures thereof.

The additional fungicide(s) used in the composition and/or coating of the present invention is preferably soluble in the liquid vehicle.

In one aspect the present invention provides a marine anti-foulant consisting of a composition as defined above.

Preferably, the anti-foulant is self-polishable.

In one aspect of the present invention, the substrate or substrate generator and/or the enzyme are encapsulated. Preferably, the substrate/substrate generator and/or enzyme are encapsulated by a semi-permeable membrane.

The substrate/substrate generator and enzyme may be encapsulated individually independently of each other or may be encapsulated together. In the former embodiment, the substrate/substrate generator or enzyme may be activated by the foulant. For example, the encapsulating material may be selected such that on contact with a foulant, the substrate/substrate generator or enzyme may be released to contact the other of the substrate/substrate generator or enzyme. In this way, a composition may be provided which only provides an anti-foulant compound or increases provision of an anti-foulant compound when contacted with a foulant.

The composition of the present invention can be provided as a ready-for-use product or as a concentrate. The ready-for-use product may be in the form of an aqueous solution, aqueous dispersion, oil solution, oil dispersion, emulsion, or an aerosol preparation. The concentrate can be used, for example, as an additive for coating, or can be diluted prior to use with additional solvents or suspending agents.

An aerosol preparation according to the invention may be obtained in the usual manner by incorporating the composition of the present invention comprising or dissolved or suspended in, a suitable solvent, in a volatile liquid suitable for use as a propellant, for example the mixture of chlorine and fluorine derivatives of methane and ethane commercially available under the trademark "Freon", or compressed air.

As discussed in US-A-5071479 the composition and/or coating of the present invention may include additional ingredients known to be useful in preservatives and/or coatings. Such ingredients include fixatives such as carboxymethylcellulose, polyvinyl alcohol, paraffin, co-solvents, such as ethylglycol acetate and methoxypropyl acetate, plasticisers such as benzoic acid esters and phthlates, e.g., dibutyl phthalate, dioctyl phthalate and didodecyl phthalate, derivatives and mixtures thereof. Optionally dyes, color pigments, corrosion inhibitors, chemical stabilizers or siccatives (dryers) such as cobalt octate and cobalt naphthenate, may also be included depending on specific applications.

The composition and/or coating of the present invention can be applied by any of the techniques known in the art including brushing, spraying, roll coating, dipping and combinations thereof.

Compositions of the present invention can be prepared simply by mixing the various ingredients at a temperature at which they are not adversely affected. Preparation conditions are not critical. Equipment and methods conventionally employed in the manufacture of coating and similar compositions can be advantageously employed.

The invention will now be described, by way of example only, in the following Examples.

### EXAMPLES

The anti-fouling effect of an anti-fouling composition of the present invention is tested according to the following examples. These Examples show the effectiveness of the present composition at preventing fouling. The Examples also provide for the optimisation of the anti-fouling properties of the present composition.

The hexose oxidase (HOX) used in each of the present examples is available from DaniscoCultor. The HOX is a fermented product is from yeast *Hansenula polymorpha* expressing the gene encoding the HOX enzyme cloned from the marine algae *Chondrus crispus.*

### EXAMPLE 1 - Preparation Of An Anti-fouling Composition ("One-Step")

Soluble or immobilised hexose oxidase or another hydrogen peroxide generating enzyme such as glucose oxidase is tested as a anti-foulant compound generating enzyme in an anti-fouling composition. The hexose oxidase may be immobilised for example by binding to an anion exchanger, Q Sepharose FF™ (available from Pharmacia) using 20 mM triethanolamine buffer, pH 7.3. Alternatively, hexose oxidase or alternative hydrogen peroxide generating enzymes is covalently linked to a suitable carrier such as epoxy activated Sepharose™ (Pharmacia, Sweden), carbodiimide activated agarose (Bio-Rad, USA). Other conventional procedures known in the art for immobilisation may also be utilised

The range of concentrations used is 0.0001 to 1000 U of hexose oxidase activity/hydrogen peroxide generating enzyme per ml of anti-fouling composition. One unit of enzyme activity is defined as the amount of enzyme which produces 1 µmol of H₂O₂ per min at 25 °C.

To ascertain its suitability for use in the present invention the activity of the enzyme may be assayed as follows. Hexose oxidase (HOX) activity is measured in accordance with the following procedure.

The HOX assay is based on the measurement of hydrogen peroxide generated in the oxidation of glucose. The hydrogen peroxide oxidises *o*-dianisidine in the presence of peroxidase to form a dye.

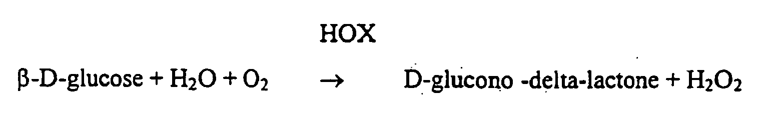

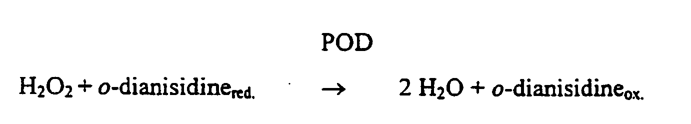

### Reagents

1. 100 mM phosphate buffer, pH 6.3
2. 100 mM D-glucose (SIGMA, G-8270) in 100 mM phosphate buffer, pH 6.3
*3. o*-Dianisidine (SIGMA, D-3252), 3.0 mg/ml in distilled water
4. Peroxidase (SIGMA, P-8125), 0.10 mg/ml in 100 mM phosphate buffer, pH 6.3

### Assay

120 µl reagent 1
150 µl reagent 2
10 µl reagent 3
10 µl reagent 4 and
10 µl enzyme solution

The assay is performed in a microtiter plate. The reaction is initiated by the addition of enzyme solution. The mixture is incubated at 25°C for 15 min with shaking. The blank run contains all the components with water instead of enzyme solution. The formation of the dye is measured in a microtiter plate reader at 405 nm. The linearity of the reaction can be checked by using a kinetics programme on the microplate reader.

A hydrogen peroxide standard curve can be constructed by using varying concentrations of fresh H₂O₂ (MERCK).

### EXAMPLE 2 - Preparation Of An Anti-fouling Composition ("Two-Step")

Glucose and galactose in concentrations of 0.01 to 100 µg per ml of anti-fouling composition are tested as substrates to generate a substrate for hexose oxidase in the systems described in Example 1. In order to provide a continuous substrate generating system, starch, preferably intact starch granules from wheat, maize or potato, in a concentration of from 0.01 ng to 100 µg per ml of anti-fouling composition, are used together with amyloglucosidase (GRINDAMYL™ AG 1500 Bakery Enzyme from DaniscoCultor or another commercial amyloglucosidase product). The components are present in concentrations providing from 0.000001 to 10 AGU per ml of anti-fouling composition.

1 AGU is defined as the amyloglucosidase activity which releases 1 µmol of glucose per minute from maltose (0.5% w/v) in 50 mM sodium acetate, pH 5.0 (adjusted with concentrated acetic acid) at 40°C. The assay is stopped by transferring 200 µl of assay mix to 100 µl of 0.1 M hydrochloric acid chloride and the amount of glucose released is measured using glucose dehydrogenase reagent (Merck no. 12193) or another glucose detection system.

### EXAMPLE 3 - Generation of hydrogen peroxide by paint containing HOX

In order to test the ability of hexose oxidase (HOX) to generate hydrogen peroxide the following experiment was performed.

To 11.0 g of paint (water-based wall painting Sadolin Glans 7 and oil based Histor 9010, respectively) were added 0.2, 0.5 and I g, respectively, of HOX (DaniscoCultor fermented product from *Hansenula polymorpha*) spraydried on starch (10 U/g). To the water based paint was also added 5g of water per treatment.

Disposable plastic transfer pipettes (Sarstedt) were dipped (head part) in the paint. The transfer pipettes were left to air dry for 3 hours.

Hexose oxidase (HOX) activity was then measured by immersion of the paint covered pipette head into a glass tube with 2 mL of HOX assay reagent, see below, the only HOX activity coming from the HOX in the paint.

The tubes were incubated at room temperature.

As a blank was used paint without added HOX.

The result of the experiment is shown in table 1. HOX is homogeneously distributed in the paint, since the whole surface of the paint immediately turns red when it gets in contact with the HOX assay reagent. The colour development is observed immediately indicating that the paint does not have any inhibiting effect on the HOX activity. This experiment proves that HOX is able to generate hydrogen peroxide from exogenous added substrate (here glucose) even when immobilised in a paint matrix after drying.

**Table 1**

| | Activity |
|---|---|
| Water based paint, blank | 0 |
| 0.2g HOX | + |
| 0.5g HOX | ++ |
| 1.0g HOX | +++ |
| Oil based paint, blank | 0 |
| 0.2g HOX | + |
| 0.5g HOX | ++ |
| 1.0g HOX | +++ |
| Control, assay reagent plus free HOX | +++ |

The range of concentrations used is 0.0001 to 1000 U of hexose oxidase activity or of an alternative hydrogen peroxide generating enzyme per ml of antifouling composition.

### EXAMPLE 4 - Model System For Coating

Dialysis tubing containing an anti-fouling composition is used as a model system for a coating to prevent fouling on the surface of a coated material.

An anti-fouling composition within the dialysis tubing is used to generate an concentration of hydrogen peroxide on the surface of the dialysis tubing effective to prevent fouling. The dialysis tubing used has a cut off value of about 10000 Da. The dialysis tubing is either dialysis tubing or a dialysis cassette (such as Slide-A-Lyzer™ available from Pierce; IL, USA).

The dialysis tubing is immersed in a glass beaker with 1 to 5 litre of lake or sea water collected as described above. The glass beaker is stirred slowly with a magnetic stirrer and incubated at room temperature in proximity to a window to allow daylight to fall thereon. Fouling on the dialysis tubing is monitored visually for up to 4 weeks based on the appearance of a microbial growth layer on the dialysis tubing and rated on a scale of 1 to 5 as described above. As negative control a dialysis tube containing tap water is used.

Optionally, catalase immobilised onto nitrocellulose membrane pieces, which have subsequently been blocked with 0.1% Tween 20, are added to the lake or sea water in order to avoid accumulation of hydrogen peroxide in the water surrounding the dialysis tubing. The concentration of catalase used is in the range of 0.000001 to 100 CU, where 1 CU is defined as the catalase activity degrading 1 µmol of hydrogen peroxide per minute at 30°C in 50 mM sodium phosphate buffer, pH 7.0, as described for catalase in the Sigma catalogue: Biochemicals Organic Compounds for Research and Diagnostic Reagents, Sigma Chemical Company 1995, page 221.

The compositions of the present invention are effective at preventing fouling.

### EXAMPLE 5 - Stability of HOX in paint

The painted heads of transfer pipettes described in example 1 were kept at room temperature for 2 month and were then "assayed" in reagent mix as described in example 2.

**Table 2**

| | Activity |
|---|---|
| Water based paint, blank | 0 |
| 0.2g HOX | + |
| 0.5g HOX | ++ |
| 1.0g HOX | not determined |
| Oil based paint, blank | 0 |
| 0.2g HOX | +++ |
| 0.5g HOX | +++ |
| 1.0g HOX | +++ |
| Control, assay reagent plus free HOX | not determined |

From the results in table 2 it is clear that HOX was stable for two month at room temperature in a dry paint matrix.

### EXAMPLE 6 -Testing Of Coating

### Set Up Of A Test System For An Anti-fouling Composition

0.5 to 5 ml. samples of lake or sea water were collected in test tubes from the lake Brabrandsøen near Aarhus, Denmark, and from the Baltic sea off Aarhus. On the day of collection of the water samples the anti-fouling composition to be tested is added to the test tubes and they are sealed with Parafilm™.

The test tubes are incubated at room temperature in proximity to a window to allow daylight to fall thereon. Fouling is monitored visually for up to 4 weeks based on the appearance of a microbial growth layer on the walls of the test tube. For comparison a test tube with 0.1% of sodium azide and a test tube without anti-fouling composition are used as positive and negative controls, respectively.

These test tubes are rated 1 and 5, respectively, on a scale of 1 to 5 for highly efficient to no anti-fouling activity, respectively.

Commercial marine anti-fouling coating material without added anti-fouling biocide is used. Anti-fouling compositions according to the present invention are mixed into the coating material and applied to the surface of metal, glass and plastic plates according to the instructions of the manufacturer of the coating material.

Coated plates are immersed into water in a lake or in sea water. Fouling on the plates is monitored visually for up to 2 years based on the appearance of a microbial growth layer on the plates and rated on a scale of 1 to 5 as described above. As negative control a coating without anti-fouling composition is used.

The compositions of the present invention are effective at preventing fouling.

### EXAMPLE 7 - Stability of antifouling composition in aquarium water

To 10.0 g of paint (oil based Histor 9010) were added 500 mg of starch (Merck 1253), 50 mg of HOX (DaniscoCultor fermented product from *Hansenula polymorpha*) spraydryed on starch (10 U/g).

A disposable plastic transfer pipette (Sarstedt) was dipped (head part) in the paint. The transfer pipette was left to air dry for 24 hours. It was then kept in 250 mL of water from an aquarium in a Kautex bottle for two month. The bottle was standing in a window in full daylight. After two month the pipette head was washed, airdried and then "assayed" in complete HOX reagent mix. The HOX still showed full activity.

### EXAMPLE 8 - Proof of substrate generating concept

In order to provide a continuous substrate generating system starch, preferably intact starch granules from wheat, maize or potato in a concentration of from 0.01 ng to 100 mg per ml of antifouling composition, as well as amyloglucosidase (AMG)(GRINDAMYL™ AG 10000 Bakery Enzyme from DaniscoCultor or another commercial amyloglucosidase product) in concentrations providing from 0.000001 to 100 AGU per ml of antifouling composition are used together with HOX.

To 10.0 g of paint (oil-based Histor 9010) were added 500 mg of starch (Merck), HOX (DaniscoCultor fermented product from *Hansenula polymorpha*) spraydried on starch (10 U/g) and AMG (10000 AGU/g) as indicated in the table.

Disposable plastic transfer pipettes (Sarstedt) were dipped (head part) in the paint. The transfer pipettes were left to air dry for 3 hours.

Hexose oxidase (HOX) activity was then measured by immersion of the paint covered pipette head into a glasstube with 2 mL of HOX assay reagent without glucose, for assay reagent see example 1, the only HOX activity coming from the HOX in the paint and the only substrate for HOX generated by AMG in the paint by hydrolysing starch in the paint to glucose.

The tubes were incubated at room temperature for 48 hours.

As a blank was used paint without HOX and AMG added.

**Table 3**

| | Activity |
|---|---|
| 50 mg HOX + 10 mg AMG | + |
| 50 mg HOX + 20 mg AMG | + |
| 50 mg HOX | - |
| Blank (no enzymes) | - |

The results given in table 3 shows that the combination of HOX and AMG works as intended. AMG is generating glucose from the co-immobilised starch in the paint and HOX is generating hydrogen peroxide from the generated glucose.

### EXAMPLE 9 - Temperature Activity

Hexose oxidase (purified HOX) was evaluated with regard to activity as a function of temperature and compared with a commercial glucose oxidase (Amano 081443/00018)

### Procedure:

Sample: The enzyme sample is dissolved in water and desalted on a PD10
column using 20mM phosphate buffer pH 6.3 and diluted to 0.4 U/ml

To an Elisa well is added:
150 µl 100 mM glucose in 100 mM phosphate buffer, pH 6.3
120 µl 100 mM phosphate buffer, pH 6.3
10 µl o-dianisidine (3 mg/ml in water)
10 µl peroxidase (0,10 mg/ml in 100 mM phosphate buffer, pH 6.3)
10 µl Sample
Assayed 10 min at 30 °C and measured at 405 nm.

### Results

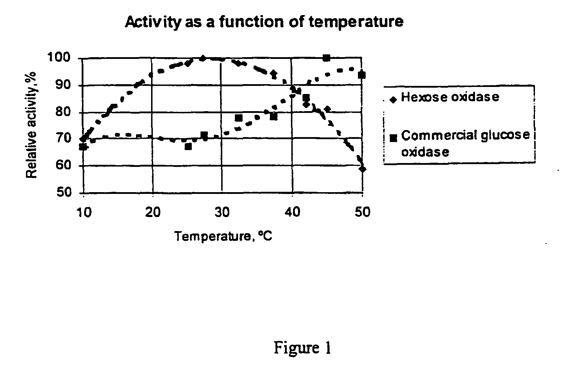

The results from the activity measurement as a function of temperature is shown in table 4 and fig. 1

**Table 4**

| Temperature, | Hexose oxidase | Commercial glucose oxidase |
|---|---|---|
| °C | Relative activity, % | Relative activity, % |
| 10 | 70 | 67 |
| 25 | 98 | 67 |
| 27.5 | 100 | 72 |
| 32.5 | 98 | 78 |
| 37.5 | 94 | 78 |
| 42 | 83 | 85 |
| 45 | 81 | 100 |
| 50 | 59 | 94 |

The results from the activity versus temperature clearly illustrate a difference in the activity profile.

Hexose oxidase has its optimum temperature between 25-35°C, which is almost coinciding with the optimum for the maximum fouling temperature. On the contrary it is seen that GOX has an optimum temperature at 50°C which is far above the temperatures that can ever be reached at sea.

## Claims

1. An anti-fouling composition comprising
(i) a surface coating material;
(ii) a first enzyme and a first substrate, wherein said first substrate is an oligomer or a polymer of a second substrate, said second substrate being a substrate for an oxidative enzyme, and wherein said first enzyme is capable of generating said second substrate from said first substrate; and
(iii) a second enzyme, wherein said second enzyme is an oxidase; and wherein said second enzyme generates an anti-fouling compound when acting on said second substrate.

2. A composition according to claim 1 wherein the second enzyme is selected from glucose oxidase, L amino acid oxidase, D amino oxidase, galactose oxidase, hexose oxidase, pyranose oxidase, malate oxidase, cholesterol oxidase, arylalcohol oxidase, alcohol oxidase, lathosterol oxidase, aspartate oxidase, amine oxidase, D glutamate oxidase, ethanolamine oxidase, NADH oxidase, urate oxidase (uricase) and mixtures thereof.

3. A composition according to any one of the preceding claims wherein the enzyme is hexose oxidase.

4. A composition according to claim 3, wherein the hexose oxidase is obtained by cloning and expression in recombinant host organisms of a gene encoding the protein.

5. A composition according to any one of the preceding claims wherein the second substrate is a sugar.

6. A composition according to claim 4 wherein the sugar is glucose.

7. A composition according to any one of the preceding claims wherein the first enzyme is amyloglucosidase.

8. A composition according to any one of the preceding claims wherein the first substrate is selected from starch, lactose, cellulose, dextrose, peptide, inulin, and mixtures thereof

9. A composition according to any one of the preceding claims wherein the first substrate is starch.

10. A composition according to any one of the preceding claims wherein the composition further comprises a binder to immobilise at least one of the constituents of the composition, preferably to immobilise the enzyme.

11. A composition according to any one of the preceding claims wherein the composition is formulated as a coating, a lacquer, a stain or an enamel.

12. A composition according to any one of the preceding claims wherein the composition further comprises a surface coating material selected from polyvinyl chloride resins in a solvent based system, chlorinated rubbers in a solvent based system, acrylic resins and methacrylate resins in solvent based or aqueous systems, vinyl chloride-vinyl acetate copolymer systems as aqueous dispersions or solvent based systems, butadiene copolymers, butadiene-styrene rubbers, butadiene-acrylonitrile rubbers, butadiene-styrene-acrylonitrile rubbers, drying oils, linseed oil, alkyd resins, asphalt, epoxy resins, urethane resins, polyester resins, phenolic resins, derivatives and mixtures thereof.

13. A coating consisting of a composition according to any one of the preceding claims.

14. A coating according to claim 13 formulated for treatment of a surface selected from outdoor wood work, external surface of a central heating system, and a hull of a marine vessel.

15. A marine anti-foul consisting of a composition according to any one of the preceding claims.

16. A marine anti-foul according to claim 15 wherein the anti-foulant is self-polishable.

17. A method for releasing an anti-fouling compound from a surface coating, which method comprises incorporating in a surface coating:
(i) a first enzyme and a first substrate, wherein said first substrate is an oligomer or a polymer of a second substrate, said second substrate being a substrate for an oxidase enzyme, and wherein said first enzyme generates said second substrate from said first substrate;
(ii) a second enzyme, wherein said second enzyme is an oxidase; and wherein the second enzyme generates an anti-fouling compound by acting on said second substrate.

## Patentansprüche

1. Bewuchsverhindernde Zusammensetzung, umfassen
(i) ein Oberflächenbeschichtungsmaterial
(ii) erster Enzym und ein erstes Substrat, wobei das erste Substrat ein Oligomer oder ein Polymer eines zweiten Substrats ist, wobei das zweite Substrat ein Substrat für ein oxidatives Enzym ist, und wobei das erste Enzym in der Lage ist, das zweite Substrat aus dem ersten Substrat zu erzeugen, und
(iii) ein zweites Enzym, wobei das zweite Enzym eine Oxidase ist, und wobei das zweite Enzym eine bewuchsverhindernde Verbindung bildet, wenn es auf das zweite Substrat wirkt.

2. Zusammensetzung gemäß Anspruch 1, wobei das zweite Enzym ausgewählt ist unter Glucoseoxidase, L-Aminosäueoxidase, D-Aminooxidase, Galactoseoxidase, Hexoseoxidase, Pyranoseoxidase, Malatoxidase, Cholesteroloxidase, Arylalkoholoxidase, Alkoholoxidase, Lathosteroloxidase, Aspartatoxidase, Aminoxidase, D-Glutamatoxidase, Ethanolaminoxidase, NADH-Oxidase, Uratoxidase (Uricase) und Gemischen davon.

3. Zusammensetzung gemäß einem der vorangehenden Ansprüche, wobei das Enzym Hexoseoxidase ist.

4. Zusammensetzung gemäß Anspruch 3, wobei die Hexoseoxidase durch Klonierung und Expression eines das Protein codierenden Gens in rekombinanten Wirtsorganismen erhalten wird.

5. Zusammensetzung gemäß einem der vorangehenden Ansprüche, wobei das zweite Substrat ein Zucker ist.

6. Zusammensetzung gemäß Anspruch 4, wobei der Zucker Glucose ist.

7. Zusammensetzung gemäß einem der vorangehenden Ansprüche, wobei das erste Enzym Amyloglucosidase ist.

8. Zusammensetzung gemäß einem der vorangehenden Ansprüche, wobei das erste Substrat unter Stärke, Lactose, Zellulose, Dextrose, Peptid, Inulin und Gemischen davon ausgewählt ist.

9. Zusammensetzung gemäß einem der vorangehenden Ansprüche, wobei das erste Substrat Stärke ist.

10. Zusammensetzung gemäß einem der vorangehenden Ansprüche, wobei die Zusammensetzung weiter ein Bindemittel umfasst, um wenigstens einen der Bestandteile der Zusammensetzung zu immobilisierten, vorzugsweise um das Enzym zu immobilisieren.

11. Zusammensetzung gemäß einem der vorangehenden Ansprüche, wobei die Zusammensetzung als eine Beschichtung, ein Lack, ein Färbemittel oder eine Emaille formuliert ist.

12. Zusammensetzung gemäß einem der vorangehenden Ansprüche, wobei die Zusammensetzung weiter ein Oberflächenbeschichtungsmaterial umfasst, welches ausgewählt ist unter Polyvinylchloridharzen in einem System auf Lösungsmittelbasis, Chlorkautschuken in einem System auf Lösungsmittelbasis, Acrylharzen und Methacrylharzen in einem System auf Lösungsmittelbasis oder wässrigen Systemen, Vinylchlorid-Vinylacetat-Copolymersystemen als wässrige Dispersionen oder Systeme auf Lösungsmittelbasis, Butadien-Copolymeren, Butadien-Styren-Kautschuken, Butadien-Acrylnitril-Kautschuken, Butadien-Styren-Acrylnitril-Kautschuken, trocknenden Ölen, Leinöl, Alkydharzen, Asphalt, Epoxyharzen, Urethanharzen, Polyesterharzen, Phenolharzen, Derivaten und Gemischen davon.

13. Beschichtung, bestehend aus einer Zusammensetzung gemäß einem der vorangehenden Ansprüche.

14. Beschichtung gemäß Anspruch 13, die für die Behandlung einer Oberfläche, ausgewählt unter im Freien befindlichen Holzmaterial, äußeren Oberflächen eines Zentralheizungssystems und einer Oberfläche eines Seefahrzeugs, formuliert ist.

15. Meeresbewuchsschutz, bestehend aus einer Zusammensetzung gemäß einem der vorangehenden Ansprüche.

16. Meeresbewuchsschutz gemäß Anspruch 15, wobei der Bewuchsschutz selbstpolierend ist.

17. Verfahren zur Freisetzung einer bewuchsverhindernden Verbindung aus einer Oberflächenbeschichtung, wobei das Verfahren umfaßt, dass folgendes in eine Oberflächenbeschichtung ausgenommen wird:
(i) ein erstes Enzym und ein erstes Substrat, wobei das erste Substrat ein Oligomer oder ein Polymer eines zweiten Substrats ist, wobei das zweite Substrat ein Substrat für ein Oxidaseenzym ist, und wobei das erste Enzym das zweite Substrat aus dem ersten Substrat erzeugt,
(ii) ein zweites Enzym, wobei das zweite Enzym eine Oxidase ist, und wobei das zweite Enzym eine bewuchsverhindernde Verbindung erzeugt, indem es auf das zweite Substrat wirkt.

## Revendications

1. Composition antisalissure comprenant :
(i) un matériau de revêtement de surface ;
(ii) une première enzyme et un premier substrat, où ledit premier substrat est un oligomère ou un polymère d'un second substrat, ledit second substrat étant un substrat pour une enzyme oxydante et où ladite première enzyme peut générer ledit second substrat à partir dudit premier substrat ;
(iii) une seconde enzyme, où ladite seconde enzyme est une oxydase ; et où ladite seconde enzyme génère un composé antisalissure lorsqu'il agit sur ledit second substrat.

2. Composition selon la revendication 1, dans laquelle la seconde enzyme est choisie parmi la glucose oxydase, la L-acide aminé oxydase, la D-amino oxydase, la galactose oxydase, l'hexose oxydase, la pyranose oxydase, la malate oxydase, la cholestérol oxydase, l'arylalcool oxydase, l'alcool oxydase, la lathostérol oxydase, l'aspartate oxydase, l'amine oxydase, la D-glutamate oxydase, l'éthanolamine oxydase, la NADH oxydase, l'urate oxydase (uricase) et les mélanges de celles-ci.

3. Composition selon l'une quelconque des revendications précédentes, dans laquelle l'enzyme est l'hexose oxydase.

4. Composition selon la revendication 3, dans laquelle l'hexose oxydase est obtenue par clonage et expression dans des organismes hôtes recombinants d'un gène codant pour la protéine.

5. Composition selon l'une quelconque des revendications précédentes, dans laquelle le second substrat est un sucre.

6. Composition selon la revendication 4, dans laquelle le sucre est le glucose.

7. Composition selon l'une quelconque des revendications précédentes, dans laquelle la première enzyme est l'amyloglucosidase.

8. Composition selon l'une quelconque des revendications précédentes, dans laquelle le premier substrat est choisi parmi l'amidon, le lactose, la cellulose, la dextrose, un peptide, l'insuline et les mélanges de ceux-ci.

9. Composition selon l'une quelconque des revendications précédentes, dans laquelle le premier substrat est l'amidon.

10. Composition selon l'une quelconque des revendications précédentes, dans laquelle la composition comprend en outre un liant pour immobiliser l'un au moins des constituants de la composition, de préférence pour immobiliser l'enzyme.

11. Composition selon l'une quelconque des revendications précédentes, dans laquelle la composition est formulée sous forme d'un revêtement, d'une laque, d'une teinture ou d'un émail.

12. Composition selon l'une quelconque des revendications précédentes, dans laquelle la composition comprend en outre un matériau de revêtement de surface choisi parmi les résines de poly(chlorure de vinyle) dans un système à base d'un solvant, les caoutchoucs chlorés dans un système à base d'un solvant, les résines acryliques et les résines méthacryliques dans des systèmes à base d'un solvant ou aqueux, les systèmes de copolymère de chlorure de vinyle-acétate de vinyle sous forme de dispersions aqueuses ou de systèmes à base d'un solvant, les copolymères de butadiène, les caoutchoucs butadiène-styrène, les caoutchoucs butadiène-acrylonitrile, les caoutchoucs butadiène-styrène-acrylonitrile, les huiles de séchage, l'huile de lin, les résines alkydiques, l'asphalte, les résines époxy, les résines d'uréthane, les résines de polyester, les résines phénoliques, les dérivés et les mélanges de ceux-ci.

13. Revêtement constitué d'une composition selon l'une quelconque des revendications précédentes.

14. Revêtement selon la revendication 13, formulé pour le traitement d'une surface choisie parmi un ouvrage en bois extérieur, une surface extérieure d'un système de chauffage central et une coque d'un navire marin.

15. Agent antisalissure marin constitué d'une composition selon l'une quelconque des revendications précédentes.

16. Agent antisalissure marin selon la revendication 15, dans lequel l'agent antisalissure est autopolissable.

17. Procédé de libération d'un composé antisalissure depuis une revêtement de surface, procédé qui comprend l'incorporation dans un revêtement de surface :
(i) d'une première enzyme et d'un premier substrat, où ledit premier substrat est un oligomère ou une polymère d'un second substrat, ledit second substrat étant un substrat pour une enzyme oxydase et où ladite première enzyme génère ledit second substrat à partir dudit premier substrat ; et
(ii) d'une seconde enzyme, où ladite seconde enzyme est une oxydase ; et où ladite seconde enzyme génère un composé antisalissure en agissant sur ledit second substrat.


### SEQUENCE LISTING

<110> Danisco A/S
<120> Composition
<130> P006441EPA
<140> 07001141.6
   <141> 2000-06-02
<150> GB 9913050.2
   <151> 1999-06-04
<160> 2
<170> PatentIn version 3.4
<210> 1
   <211> 1644
   <212> DNA
   <213> Chondrus crispus
<220>
   <221> CDS
   <222> (1) .. (1644)
<400> 1
   atg gct act ttg cca caa aag gac cca ggt tac att gtt att gac gtc       48
   Met Ala Thr Leu Pro Gln Lys Asp Pro Gly Tyr Ile Val Ile Asp Val
   1               5                   10                  15
   aac gct ggt act cca gac aag cct gac cca aga ttg cca tcc atg aag       96
   Asn Ala Gly Thr Pro Asp Lys Pro Asp Pro Arg Leu Pro Ser Met Lys
               20                  25                  30
   caa ggt ttc aac aga aga tgg att ggt acc aac atc gat ttc gtt tac      144
   Gln Gly Phe Asn Arg Arg Trp Ile Gly Thr Asn Ile Asp Phe Val Tyr
           35                  40                  45
   gtc gtt tac act cca caa ggt gct tgt act gct ttg gac aga gct atg      192
   Val Val Tyr Thr Pro Gln Gly Ala Cys Thr Ala Leu Asp Arg Ala Met
       50                  55                  60
   gaa aag tgt tct cca ggt acc gtc aga atc gtt tct ggt ggt cac tgt      240
   Glu Lys Cys Ser Pro Gly Thr Val Arg Ile Val Ser Gly Gly His Cys
   65                  70                  75                  80
   tac gaa gac ttc gtt ttc gac gaa tgt gtc aag gct att atc aac gtt      288
   Tyr Glu Asp Phe Val Phe Asp Glu Cys Val Lys Ala Ile Ile Asn Val
                   85                  90                  95
   act ggt ttg gtt gaa tct ggt tac gac gac gat aga ggt tac ttc gtc      336
   Thr Gly Leu Val Glu Ser Gly Tyr Asp Asp Asp Arg Gly Tyr Phe Val
               100                 105                 110
   tct tcc ggt gac acc aac tgg ggt tcc ttc aag acc ttg ttc aga gac      384
   Ser Ser Gly Asp Thr Asn Trp Gly Ser Phe Lys Thr Leu Phe Arg Asp
           115                 120                 125
   cac ggt aga gtt ttg cca ggt ggt tcc tgt tac tcc gtc ggt ttg ggt      432
   His Gly Arg Val Leu Pro Gly Gly Ser Cys Tyr Ser Val Gly Leu Gly
       130                 135                 140
   ggt cac att gtc ggt gga ggt gac ggt att ttg gcc aga ttg cac ggt      480
   Gly His Ile Val Gly Gly Gly Asp Gly Ile Leu Ala Arg Leu His Gly
   145                 150                 155                 160
   ttg cca gtc gat tgg tta tcc ggt gtt gaa gtt gtc gtt aag cca gtc      528
   Leu Pro Val Asp Trp Leu Ser Gly Val Glu Val Val Val Lys Pro Val
                   165                 170                 175
   ttg acc gaa gac tct gtt ctt aag tac gtt cac aag gat tcc gaa ggt      576
   Leu Thr Glu Asp Ser Val Leu Lys Tyr Val His Lys Asp Ser Glu Gly
               180                 185                 190
   aac gac ggt gag ttg ttt tgg gct cac act ggt gga ggt gga ggt aac      624
   Asn Asp Gly Glu Leu Phe Trp Ala His Thr Gly Gly Gly Gly Gly Asn
           195                 200                 205
   ttc ggt att atc acc aaa tac tac ttc aag gat ttg cca atg tct cca      672
   Phe Gly Ile Ile Thr Lys Tyr Tyr Phe Lys Asp Leu Pro Met Ser Pro
       210                 215                 220
   aga ggt gtc atc gct tct aac tta cac ttc tct tgg gac ggt ttc act      720
   Arg Gly Val Ile Ala Ser Asn Leu His Phe Ser Trp Asp Gly Phe Thr
   225                 230                 235                 240
   aga gat gcc ttg caa gat ttg ttg act aag tac ttc aag ttg gct aga      768
   Arg Asp Ala Leu Gln Asp Leu Leu Thr Lys Tyr Phe Lys Leu Ala Arg
                   245                 250                 255
   tgt gat tgg aag aat act gtt ggt aag ttc caa atc ttc cac caa gca      816
   Cys Asp Trp Lys Asn Thr Val Gly Lys Phe Gln Ile Phe His Gln Ala
               260                 265                 270
   gct gaa gag ttt gtt atg tac ttg tat aca tcc tac tct aac gac gcc      864
   Ala Glu Glu Phe Val Met Tyr Leu Tyr Thr Ser Tyr Ser Asn Asp Ala
           275                 280                 285
   gag aga gaa gtt gcc caa gac aga cac tat cat ttg gag gct gac att      912
   Glu Arg Glu Val Ala Gln Asp Arg His Tyr His Leu Glu Ala Asp Ile
       290                 295                 300
   gaa cag atc tac aaa aca tgc gag cct acc aaa gct ctt ggt ggt cat      960
   Glu Gln Ile Tyr Lys Thr Cys Glu Pro Thr Lys Ala Leu Gly Gly His
   305                 310                 315                 320
   gct ggt tgg gct cct ttc cct gtt aga cct aga aag aga cac aca tcc     1008
   Ala Gly Trp Ala Pro Phe Pro Val Arg Pro Arg Lys Arg His Thr Ser
                   325                 330                 335
   aag act tct tat atg cat gac gag act atg gac tac cct ttc tac gct     1056
   Lys Thr Ser Tyr Met His Asp Glu Thr Met Asp Tyr Pro Phe Tyr Ala
               340                 345                 350
   ttg act gag act atc aac ggt tcc ggt cct aat cag aga ggt aag tac     1104
   Leu Thr Glu Thr Ile Asn Gly Ser Gly Pro Asn Gln Arg Gly Lys Tyr
           355                 360                 365
   aag tct gct tac atg atc aag gac ttt cca gac ttc cag att gat gtt     1152
   Lys Ser Ala Tyr Met Ile Lys Asp Phe Pro Asp Phe Gln Ile Asp Val
       370                 375                 380
   atc tgg aaa tac ctt act gag gtt cct gac ggt ttg act agt gcc gaa     1200
   Ile Trp Lys Tyr Leu Thr Glu Val Pro Asp Gly Leu Thr Ser Ala Glu
   385                 390                 395                 400
   atg aag gat gct ctt ctt cag gtt gat atg ttc ggt ggt gag att cac      1248
   Met Lys Asp Ala Leu Leu Gln Val Asp Met Phe Gly Gly Glu Ile His
                   405                 410                 415
   aag gtt gtt tgg gat gct act gca gtt gct cag aga gag tac atc atc      1296
   Lys Val Val Trp Asp Ala Thr Ala Val Ala Gln Arg Glu Tyr Ile Ile
               420                 425                 430
   aaa ctg cag tac cag aca tac tgg cag gaa gaa gac aag gat gca gtt      1344
   Lys Leu Gln Tyr Gln Thr Tyr Trp Gln Glu Glu Asp Lys Asp Ala Val
           435                 440                 445
   aac ttg aag tgg att aga gac ttt tac gag gag atg tat gag cct tat      1392
   Asn Leu Lys Trp Ile Arg Asp Phe Tyr Glu Glu Met Tyr Glu Pro Tyr
       450                 455                 460
   ggt ggt gtt cca gac cct aac act cag gtt gag agt ggt aaa ggt gtt      1440
   Gly Gly Val Pro Asp Pro Asn Thr Gln Val Glu Ser Gly Lys Gly Val
   465                 470                 475                 480
   ttt gag gga tgc tac ttc aac tac cct gat gtt gac ttg aac aac tgg      1488
   Phe Glu Gly Cys Tyr Phe Asn Tyr Pro Asp Val Asp Leu Asn Asn Trp
                   485                 490                 495
   aag aac ggt aag tat ggt gcc ttg gaa ctt tac ttt ttg ggt aac ctg      1536
   Lys Asn Gly Lys Tyr Gly Ala Leu Glu Leu Tyr Phe Leu Gly Asn Leu
               500                 505                 510
   aac aga ttg atc aag gcc aaa tgg ttg tgg gat cct aac gag atc ttc      1584
   Asn Arg Leu Ile Lys Ala Lys Trp Leu Trp Asp Pro Asn Glu Ile Phe
           515                 520                 525
   aca aac aaa cag tct atc cct act aaa cct ctt aag gag cct aag cag      1632
   Thr Asn Lys Gln Ser Ile Pro Thr Lys Pro Leu Lys Glu Pro Lys Gln
       530                 535                 540
   act aaa tag tag                                                      1644
   Thr Lys
   545
<210> 2
   <211> 546
   <212> PRT
   <213> Chondrus crispus
<400> 2
   Met Ala Thr Leu Pro Gln Lys Asp Pro Gly Tyr Ile Val Ile Asp Val
   1               5                   10                  15
   Asn Ala Gly Thr Pro Asp Lys Pro Asp Pro Arg Leu Pro Ser Met Lys
               20                  25                  30
   Gln Gly Phe Asn Arg Arg Trp Ile Gly Thr Asn Ile Asp Phe Val Tyr
           35                  40                  45
   Val Val Tyr Thr Pro Gln Gly Ala Cys Thr Ala Leu Asp Arg Ala Met
       50                  55                  60
   Glu Lys Cys Ser Pro Gly Thr Val Arg Ile Val Ser Gly Gly His Cys
   65 70 75 80
   Tyr Glu Asp Phe Val Phe Asp Glu Cys Val Lys Ala Ile Ile Asn Val
   85 90 95
   Thr Gly Leu Val Glu Ser Gly Tyr Asp Asp Asp Arg Gly Tyr Phe Val
   100 105 110
   Ser Ser Gly Asp Thr Asn Trp Gly Ser Phe Lys Thr Leu Phe Arg Asp
   115 120 125
   His Gly Arg Val Leu Pro Gly Gly Ser Cys Tyr Ser Val Gly Leu Gly
   130 135 140
   Gly His Ile Val Gly Gly Gly Asp Gly Ile Leu Ala Arg Leu His Gly
   145 150 155 160
   Leu Pro Val Asp Trp Leu Ser Gly Val Glu Val Val Val Lys Pro Val
   165 170 175
   Leu Thr Glu Asp Ser Val Leu Lys Tyr Val His Lys Asp Ser Glu Gly
   180 185 190
   Asn Asp Gly Glu Leu Phe Trp Ala His Thr Gly Gly Gly Gly Gly Asn
   195 200 205
   Phe Gly Ile Ile Thr Lys Tyr Tyr Phe Lys Asp Leu Pro Met Ser Pro
   210 215 220
   Arg Gly Val Ile Ala Ser Asn Leu His Phe Ser Trp Asp Gly Phe Thr
   225 230 235 240
   Arg Asp Ala Leu Gln Asp Leu Leu Thr Lys Tyr Phe Lys Leu Ala Arg
   245 250 255
   Cys Asp Trp Lys Asn Thr Val Gly Lys Phe Gln Ile Phe His Gln Ala
   260 265 270
   Ala Glu Glu Phe Val Met Tyr Leu Tyr Thr Ser Tyr Ser Asn Asp Ala
   275 280 285
   Glu Arg Glu Val Ala Gln Asp Arg His Tyr His Leu Glu Ala Asp Ile
   290 295 300
   Glu Gln Ile Tyr Lys Thr Cys Glu Pro Thr Lys Ala Leu Gly Gly His
   305 310 315 320
   Ala Gly Trp Ala Pro Phe Pro Val Arg Pro Arg Lys Arg His Thr Ser
                   325                 330                 335
   Lys Thr Ser Tyr Met His Asp Glu Thr Met Asp Tyr Pro Phe Tyr Ala
               340                 345                 350
   Leu Thr Glu Thr Ile Asn Gly Ser Gly Pro Asn Gln Arg Gly Lys Tyr
           355                 360                 365
   Lys Ser Ala Tyr Met Ile Lys Asp Phe Pro Asp Phe Gln Ile Asp Val
       370                 375                 380
   Ile Trp Lys Tyr Leu Thr Glu Val Pro Asp Gly Leu Thr Ser Ala Glu
   385                 390                 395                 400
   Met Lys Asp Ala Leu Leu Gln Val Asp Met Phe Gly Gly Glu Ile His
                   405                 410                 415
   Lys Val Val Trp Asp Ala Thr Ala Val Ala Gln Arg Glu Tyr Ile Ile
               420                 425                 430
   Lys Leu Gln Tyr Gln Thr Tyr Trp Gln Glu Glu Asp Lys Asp Ala Val
           435                 440                 445
   Asn Leu Lys Trp Ile Arg Asp Phe Tyr Glu Glu Met Tyr Glu Pro Tyr
       450                 455                 460
   Gly Gly Val Pro Asp Pro Asn Thr Gln Val Glu Ser Gly Lys Gly Val
   465                 470                 475                 480
   Phe Glu Gly Cys Tyr Phe Asn Tyr Pro Asp Val Asp Leu Asn Asn Trp
                   485                 490                 495
   Lys Asn Gly Lys Tyr Gly Ala Leu Glu Leu Tyr Phe Leu Gly Asn Leu
               500                 505                 510
   Asn Arg Leu Ile Lys Ala Lys Trp Leu Trp Asp Pro Asn Glu Ile Phe
           515                 520                 525
   Thr Asn Lys Gln Ser Ile Pro Thr Lys Pro Leu Lys Glu Pro Lys Gln
       530                 535                 540
   Thr Lys
   545